## Europäisches Patentamt
## European Patent Office
## Office européen des brevets

(11) Publication number: **0 073 545**
**B1**

(12) **EUROPEAN PATENT SPECIFICATION**

(45) Date of publication of patent specification: **30.01.85**

(51) Int. Cl.[4]: **C 07 C 59/68,** C 07 C 51/235, C 07 C 59/125, C 08 G 65/32

(21) Application number: **82201056.7**

(22) Date of filing: **25.08.82**

(54) Process for the preparation of ethercarboxylates.

(30) Priority: **29.08.81 NL 8104025**

(43) Date of publication of application:
**09.03.83 Bulletin 83/10**

(45) Publication of the grant of the patent:
**30.01.85 Bulletin 85/05**

(84) Designated Contracting States:
**AT BE CH DE FR GB IT LI NL SE**

(56) References cited:
**FR-A-2 391 185**
**US-A-4 052 336**

**CHEMICAL ABSTRACTS, vol. 90, no. 14, April 1979, pages 23, no. 104683z, Columbus Ohio (USA);**

**CHEMICAL ABSTRACTS, vol. 90, no. 1, 1st January 1979, page 543, no. 5916w, Columbus Ohio (USA);**

**PATENTS ABSTRACTS OF JAPAN, vol. 3, no. 100(C-56), 24th August 1979, page 113;**

(73) Proprietor: **STAMICARBON B.V.**
**Postbus 10**
**NL-6160 MC Geleen (NL)**

(72) Inventor: **De Man, Hendrikus Cornelis J.**
**Irenelaan 22**
**NL-6165 CP Geleen (NL)**
Inventor: **Spronken, Josephus Marie Hubertus**
**Pastoorslaan 20**
**NL-6241 BK Bunde (NL)**

(74) Representative: **Hoogstraten, Willem Cornelis Roeland et al**
**OCTROOIBUREAU DSM Postbus 9**
**NL-6160 MA Geleen (NL)**

## Description

The invention relates to a process for the preparation of ethercarboxylates by oxidation of etheralcohols, having the general formula of $R-O-(CH_2CH_2O)_nH$, where R represents an alkyl group having 8—22 carbon atoms, a phenylgroup or an alkylphenyl group and n a whole number from 1—100, or mixtures of these etheralcohols, or by oxidation of oxyethylene-oxypropylene block copolymers having an average molecular weight of 1000—12000 and a content of 10—80% by weight ethylene oxide units, or mixtures of these copolymers.

This process, known from the United States patent specification 4,214,101, is performed at a pH of 8—13 with a molecular oxygen-containing gas and a palladium catalyst on activated carbon as carrier.

It has now been found that catalysts also containing platinum, in addition to palladium, are substantially more active in this known oxidation.

The process according to the invention for the preparation of ethercarboxylates by oxidation of the said products with a molecular oxygen-containing gas at a pH of 8—13 while using a palladium catalyst on activated carbon as carrier is characterized in that a catalyst is used which, in addition to palladium, also contains platinum in a platinum:palladium weight ratio of between 0.1:1 and 5:1.

Preference is given to performing the process according to the invention while applying a platinum:palladium weight ratio of between 0.4:1 and 2.5:1. The total quantity of platinum and palladium in the catalyst may be varied, for instance between 1 and 15% by weight, preferably between 2 and 12% by weight. Very suitable are catalysts having the platinum and palladium applied to the same quantity of activated carbon.

The process according to the invention can be performed very suitably by performing the oxidation in an aqueous medium and, by addition of, for instance, an alkalinemetalhydroxide, keeping the pH within the desired limits. In this process the chosen concentration of the starting product in the aqueous medium may vary, for instance between 5 and 50 percent by weight. Preference is given to applying a concentration of between 10 and 35 percent by weight. The reaction mixture will then be of a suitable viscosity without being diluted too much.

In the process according to the invention preference is given to the use of oxygen having a purity of at least 95% as molecular oxygen-containing gas. As the oxygen is purer the need to discharge for removing the impurities in the oxygen will be smaller and the foam formation going with this discharge will be avoided. The partial oxygen pressure may vary, for instance, between 1 and 50 bar. A partial oxygen pressure of between 1.5 and 10 bar is very suitable.

The process according to the invention may be performed at various temperatures, for instance a temperature of 50—120°C. Preferably a temperature of between 65 and 100°C is applied.

The reaction mixture obtained in the oxidation process according to the invention can be worked up in different ways, for instance by separating off the catalyst, subsequently acidifying the reaction mixture with concentrated hydrochloric acid and recovering the desired product by extraction with, for instance, chloroform or by heating the acidified reaction mixture to, for instance, 80°C with formation of two liquid layers and separating off the aqueous layer formed. If the ethercarboxylates are desired in the form of salts, the reaction mixture obtained can be worked up by just separating off the catalyst. An aqueous solution of the desired product will then be left, which solution can be concentrated, if so desired, by evaporation.

The process according to the invention is particularly suitable for the oxidation of etheralcohols or mixtures of etheralcohols having the said general formula, where R represents an alkyl group having 10—18 carbon atoms and n a whole number from 4—13.

The products obtained by applying the process according to the invention can be used in practice as surface-active substances, for instance in detergents.

In the following examples the invention will be further elucidated.

### Examples I—V

An autoclave having a capacity of 1 litre, provided with a stirrer, was filled with 5 g catalyst, 500 g distilled water and 100 g of a mixture of laurylalcoholpolyglycolethers with an average of 5.5 ethyleneoxide groups, which mixture is commercially available under the name of Akyporox RLM 55 (registered trademark).

Subsequently the air in the autoclave was replaced by oxygen (purity higher than 99%), the temperature of the mixture in the autoclave raised, during stirring, to 85°C and the pressure of the oxygen in the autoclave set at 1.5 bar. By adding aqueous sodium hydroxide (20% by weight) the pH of the reaction mixture was kept between 8 and 13, while by addition of oxygen (through a small tube having its mouth under the stirrer) the pressure in the autoclave was maintained at 1.5 bar.

Once the adsorption of oxygen by the reaction mixture had virtually ceased, the catalyst was filtered off, the reaction mixture acidified with concentrated hydrochloric acid to pH = 2 and the mixture obtained extracted three times with 100 cm$^3$ chloroform. The chloroform of the extract obtained was evaporated, and of the remaining residue the ethercarboxylic acid content (EPTON number) and the etheralcohol content (hydroxyl number) were determined. The results of these determinations were used for calculating the conversion of the starting product. The following table mentions a few data and the conversions calculated. The catalysts used, with activated carbon as carrier, had been prepared in the way described in the United States patent specification 4,052,336, which specification is hereby incorporated by reference.

For the purpose of comparison, comparative examples 1—6 are mentioned in the table. The catalyst used in example V was a mixture of the catalysts used in comparative examples 2 and 5. In the other examples the catalyst has been prepared by precipitating the platinum and palladium jointly on the activated carbon (Norit-SX-2).

It is true that in comparative examples 5 and 6 a reasonable conversion is yet achieved, but the colour of the final product is less suitable.

| Number Example or Comparative Example | Pt and /or Pd content (% wt.) in catalyst | | Duration oxidation in hours | Conversion in % | Colour product obtained |
|---|---|---|---|---|---|
| I | 2.5 % Pt | 2.5 % Pd | 6 | 88 | yellow |
| II | 5 % Pt | 5 % Pd | 6 | 89 | light yellow |
| III | 3 % Pt | 7 % Pd | 6 | 74 | yellow |
| IV | 7 % Pt | 3 % Pd | 6 | 99 | light yellow |
| V | 5 % Pt | 5 % Pd | 6 | 62 | yellow |
| 1 | | 2 % Pd | 12 | 11 | yellow |
| 2 | | 5 % Pd | 12 | 3 | yellow |
| 3 | | 10 % Pd | 24 | 4 | yellow |
| 4 | 2 % Pt | | 6 | 40 | brown |
| 5 | 5 % Pt | | 6 | 61 | reddish brown |
| 6 | 8 % Pt | | 6 | 64 | dark brown |

### Example VI

In the way described for examples I-V a mixture of nonylphenolpolyglycolethers having 9.5 ethylene oxide groups on average, which mixture is commercially available under the name of Akyporox NP 95 (registered trademark), was oxidized, using the catalyst according to example IV.

After a period of 6 hours a dark yellow product was obtained and the conversion was 100%.

### Example VII

Example IV was repeated, using as starting product a mixture of laurylalcoholpolyglycolethers having 10 ethylene oxide groups on average, which mixture is commercially available under the name of Akyporox RLM 100 (registered trademark).

After a period of 6 hours a light yellow product was obtained and the conversion was 99%.

### Claims

1. Process for the preparation of ethercarboxylates by oxidation of etheralcohols, having the general formula of $R—O—(CH_2CH_2O)_nH$, where R represents an alkyl group having 8—22 carbon atoms, a phenyl group or an alkylphenyl group and n a whole number from 1—100, or mixtures of these etheralcohols, or by oxidation of oxyethylene-oxypropylene block copolymers having an average molecular weight of 1000—12000 and a content of 10—80% by weight ethylene oxide units, or mixture of these copolymers, characterized in that a catalyst is used which, in addition to palladium, also contains platinum in a platinum:palladium weight ratio of between 0.1:1 and 5:1.

2. Process according to claim 1, characterized in that a catalyst is used with a platinum:palladium weight ratio of between 0.4:1 and 2.5:1.

3. Process according to claim 1 or 2, characterized in that a catalyst is used containing 2—12% by weight of platinum and palladium jointly.

4. Process according to any one of claims 1—3, characterized in that a catalyst is used having the platinum and palladium applied to the same quantity of activated carbon.

5. Process according to any one of claims 1—4, characterized in that the product to be oxidized is started from while being contained in an aqueous medium in a concentration of 10—35% by weight.

6. Process according to any one of claims 1—5, characterized in that the oxidation process is performed using oxygen with a partial pressure of 1.5—10 bar.

7. Process according to any one of claims 1—6, characterized in that the oxidation process is performed at a temperature of 65—100°C.

8. Process according to any one of claims 1—7, characterized in that etheralcohols or mixtures of etheralcohols having the said general formula, where R represents an alkyl group having 10—18 carbon atoms and n a whole number from 4—13, are started from.

**Revendications**

1. Procédé de préparation d'éthercarboxylates par oxydation d'éther-alcools, de formule générale $R—O—(CH_2CH_2O)_nH$, dans laquelle R représente un groupe alkyle ayant 8 à 22 atomes de carbone, un groupe phényle ou un groupe alkylphényle et n un nombre entier de 1 à 100, ou de mélanges de ces éther alcools, ou par oxydation de bloc-copolymères oxyéthylène — oxypropylène ayant un poids moléculaire moyen de 1000 à 12000 et une teneur en motifs d'oxyde d'éthylène de 10 à 80% en poids, ou de mélanges de ces copolymères, caractérisé en ce que l'on utilise un catalyseur qui, en plus du palladium, contient aussi du platine en proportion pondérale platine: palladium comprise entre 0,1:1 et 5:1.

2. Procédé selon la revendication 1, caractérisé en ce que l'on utilise un catalyseur présentant une proportion pondérale platine: palladium comprise entre 0,4:1 et 2,5:1.

3. Procédé selon la revendication 1 ou 2, caractérisé en ce que l'on utilise un catalyseur contenant 2 à 12% en poids de platine et de palladium ensemble.

4. Procédé selon l'une quelconque des revendications 1 à 3, caractérisé en ce que l'on utilise un catalyseur dont le platine et le palladium sont appliqués à la même quantité de charbon actif.

5. Procédé selon l'un quelconque des revendications 1 à 4, caractérisé en ce que l'on commence le produit à oxyder tandis qu'il est contenu dans un milieu aqueux en concentration de 10 à 35% en poids.

6. Procédé selon l'une quelconque des revendications 1 à 5, caractérisé en ce que le procédé d'oxydation est mis en oeuvre en utilisant de l'oxygène à une pression partielle de 1,5 à 10 bars.

7. Procédé selon l'une quelconque des revendications 1 à 6, caractérisé en ce que le procédé d'oxydation est mis en oeuvre à une température de 65 à 100°C.

8. Procédé selon l'une quelconque des revendications 1 à 7, caractérisé en ce que l'on part d'éther-alcools ou de mélanges d'éther-alcools possédant ladite formule générale, dans laquelle R représente un groupe alkyle ayant 10 à 18 atomes de carbone et n un nombre entier de 4 à 13.

**Patentansprüche**

1. Verfahren zur Herstellung von Äthercarboxylaten durch Oxidation von Ätheralkohlen der allgemeinen Formel $R—O—(CH_2CH_2O)_nH$, worin R eine Alkylgruppe mit 8—22 Kohlenstoffatomen, eine Phenylgruppe oder eine Alkylphenylgruppe und n eine ganze Zahl von 1—100 darstellt, oder Gemischen dieser Ätheralkohole, oder durch Oxidation von Oxyäthylen-Oxypropylen-Blockcopolymeren mit einem durchschnittlichen Molekulargewicht von 1000—12000 und einem Gehalt von 10—80 Gew.-% Äthylenoxid-Einheiten, oder Gemischen dieser Copolymeren, dadurch gekennzeichnet, daß ein Katalysator verwendet wird, der, zusätzlich zu Palladium, auch Platin in einem Gewichtsverhältnis Platin:Palladium zwischen 0,1:1 und 5:1 enthält.

2. Verfahren nach Anspruch 1, dadurch gekennzeichnet, daß ein Katalysator mit einem Gewichtsverhältnis Platin:Palladium zwischen 0,4:1 und 2,5:1 verwendet wird.

3. Verfahren nach Anspruch 1 oder 2, dadurch gekennzeichnet, daß ein Katalysator verwendet wird, der zusammen 2—12 Gew.-% Platin und Palladium enthält.

4. Verfahren nach einem der Ansprüche 1—3, dadurch gekennzeichnet, daß ein Katalysator verwendet wird, bei dem Platin und Palladium auf dieselbe Menge Aktivkohle aufgebracht sind.

5. Verfahren nach einem der Ansprüche 1—4, dadurch gekennzeichnet, daß ein wässeriges Medium, welches das zu oxidierende Produkt in einer Konzentration von 10—35 Gew.-% enthält, eingesetzt wird.

6. Verfahren nach einem der Ansprüche 1—5, dadurch gekennzeichnet, daß Oxidationsprozeß unter Verwendung von Sauerstoff mit einem Partialdruck von 1,5—10 bar durchgeführt wird.

7. Verfahren nach einem der Ansprüche 1—6, dadurch gekennzeichnet, daß der Oxidationsprozeß bei einer Temperatur von 65—100°C durchgeführt wird.

8. Verfahren nach einem der Ansprüche 1—7, dadurch gekennzeichnet, daß Ätheralkohole oder Gemische von Ätheralkoholen der genannten allgemeinen Formel, worin R eine Alkylgruppe mit 10—18 Kohlenstoffatomen und n eine ganze Zahl von 4—13 darstellt, eingesetzt werden.